# FASCICULE DE BREVET EUROPEEN

(11) **EP 1 237 526 B1**
(45) Date de publication et mention de la délivrance du brevet: **03.05.2006**
(21) Numéro de dépôt: 00979708.5
(22) Date de dépôt: 10.11.2000
(51) Int. Cl.: A61K 8/22, A61K 8/40, A61K 8/41, A61K 8/81, A61K 8/84, A61K 8/86, A61Q 5/10

(54) **COMPOSITION DE TEINTURE DIRECTE POUR FIBRES KERATINIQUES COMPRENANT UN POLYMERE EPAISSISSANT A SQUELETTE AMINOPLASTE-ETHER**
EINE ZUSAMMENSETZUNG ZUR DIREKTFÄRBUNG VON KERATINISCHEN FASERN, DIE EIN VERDICKUNGSPOLYMER MIT EINER AMINOPLASTETHER HAUPTKETTE ENTHÄLT
DIRECT DYEING COMPOSITION FOR KERATINIC FIBRES CONTAINING A THICKENING POLYMER WITH AN ETHER PLASTIC SKELETON

(30) Priorité: 08.12.1999 FR 9915486
(43) Date de publication de la demande: 11.09.2002
(73) Titulaire: L'OREAL, 75008 Paris (FR)
(72) Inventeur: ALLARD, Delphine, F-92700 Colombes (FR); LEGRAND, Frédéric, F-92400 Courbevoie (FR)
(74) Mandataire: Miszputen, Laurent
(86) Numéro de dépôt international: PCT/FR2000/003142
(87) Numéro de publication internationale: WO 2001/041718

(56) Documents cités:
- EP-A- 0 057 398
- EP-A- 0 619 111
- WO-A-99/36047

## Description

La présente invention concerne une composition de teinture directe des fibres kératiniques et en particulier des fibres kératiniques humaines telles que les cheveux, comprenant au moins un colorant direct et au moins un polymère épaississant à squelette aminoplaste-éther.

Il est connu de teindre les fibres kératiniques et en particulier les cheveux humains, avec des compositions de teinture contenant des colorants directs, en particulier des colorants benzéniques nitrés, des colorants azoïques acides, des colorants azoïques cationiques, des colorants anthraquinoniques, des colorants naturels.
Ces colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.

Pour localiser le produit de coloration à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, certains polyuréthanes, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des nuances puissantes et chromatiques de faibles sélectivités et de bonnes ténacités tout en assurant un bon état cosmétique à la chevelure traitée. Par ailleurs, elle a également constaté que les compositions tinctoriales prêtes à l'emploi contenant le ou les colorants directs, et en outre les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de teinture directe qui ne coulent pas et restent donc bien localisées au point d'application, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) tout en apportant aux cheveux de bonnes propriétés cosmétiques si on introduit (i) soit dans la composition contenant au moins un colorant direct [ou composition A1], soit (ii) dans la composition oxydante [ou composition B1] utilisée pour la coloration directe éclaircissante, ou (iii) dans les deux compositions à la fois, une quantité efficace d'un polymère à squelette aminoplaste-éther.

Ces découvertes sont à la base de la présente invention.

La présente invention a ainsi pour objet une composition de teinture directe pour fibres kératiniques, en particulier pour fibres kératiniques humaines, telles que les cheveux, comprenant, dans un milieu approprié pour la teinture, au moins un colorant direct, qui est caractérisée par le fait qu'elle contient en outre au moins un polymère à squelette aminoplaste-éther.
Un autre objet de l'invention porte sur une composition prête à l'emploi pour la teinture des fibres kératiniques qui contient au moins un colorant direct et au moins un polymère à squelette aminoplaste-éther et un agent oxydant.
Par "composition prête à l'emploi", on entend toute composition destinée à être appliquée immédiatement sur les fibres kératiniques.

L'invention vise également un procédé de teinture directe des fibres kératiniques, et en particulier des fibres kératiniques humaines telles que les cheveux, consistant à appliquer sur les fibres une composition contenant, dans un milieu approprié pour la teinture, au moins un colorant direct et au moins un polymère à squelette aminoplaste-éther.
L'invention vise aussi un procédé de teinture directe éclaircissante des fibres kératiniques, et en particulier des fibres kératiniques humaines telles que les cheveux, consistant à appliquer sur les fibres un mélange extemporané d'une composition A1 contenant, dans un milieu approprié pour la teinture, au moins un colorant direct, et d'une composition B1 contenant au moins un agent oxydant, la composition A1 et/ou la composition B1 contenant au moins un polymère à squelette aminoplaste-éther.

L'invention a également pour objet des dispositifs pour la teinture directe et la teinture directe éclaircissante des fibres kératiniques humaines ou " kits " à deux compartiments.
Un dispositif à deux compartiments pour la teinture directe selon l'invention comprend un premier compartiment renfermant, dans un milieu approprié pour la teinture, au moins un colorant direct et un deuxième compartiment renfermant au moins un polymère à squelette aminoplaste-éther.
D'autres dispositifs à 2 compartiments pour la teinture directe éclaircissante selon l'invention comprennent un compartiment qui renferme une composition A1 contenant, dans un milieu approprié pour la teinture, au moins un colorant direct, et un autre compartiment qui renferme une composition B1 contenant, dans un milieu approprié pour la teinture, un agent oxydant, au moins un polymère à squelette aminoplaste-éther étant présent dans la composition A1 ou la composition B1, ou dans chacune des compositions A1 et B1.

Mais d'autres caractéristiques, aspects, objets et avantages de l'invention apparaîtront encore plus clairement à la lecture de la description et des exemples qui suivent.

Par aminoplaste-éther, on entend au sens de la présente invention, tout produit issu de la condensation d'un aldéhyde avec une amine ou un amide.

Par aminoplaste-éther, on entend aussi au sens de la présente invention, toute unité structurale formée d'un résidu aminoplaste et d'un résidu hydrocarboné divalent lié au résidu aminoplaste par une liaison éther.

Les polymères à squelette aminoplaste-éther utilisés selon l'invention, sont choisis de préférence parmi ceux contenant au moins un motif de structure (I) suivante : 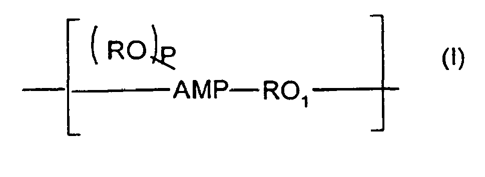 dans laquelle :
- AMP est un résidu aminoplaste avec unités alkylènes,
- R désigne un atome d'hydrogène, un radical alkyl C₁C₄ ou un radical acyle C₁C₄.
- RO₁ est un résidu alkylèneoxy divalent,
- p désigne un nombre entier positif,
le ou les groupements OR étant liés aux unités alkylènes du résidu AMP.

De préférence, les polymères à squelette aminoplaste-éther sont choisis parmi ceux contenant au moins un motif de structure (II) suivante : 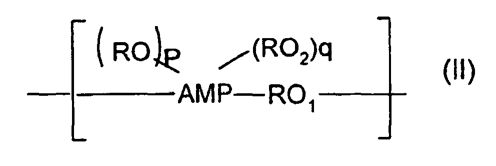 dans laquelle :
- AMP, R, RO₁ et p ont la même signification que précédemment,
- RO₂ est un groupe hydrophobe différent de RO lié à AMP via un hétéroatome et comprenant au moins deux atomes de carbone, et,
- q est un nombre entier positif.

Plus préférentiellement encore, les polymères de l'invention sont de formules (III) et (III)bis suivantes : 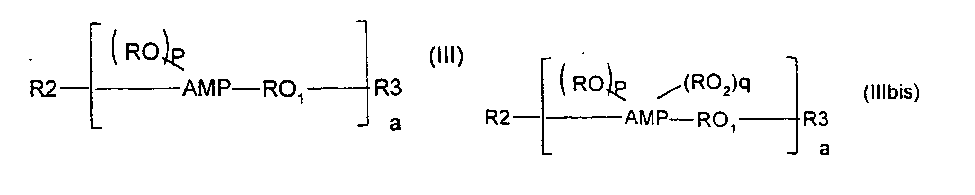 dans lesquelles :
AMP, R, RO₁, RO₂, p et q ont la même signification que précédemment, R2 ou R3, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant-un nombre plus grand que 1 et de préférence plus grand que 2.

Les résidus aminoplastes porteurs de leurs groupements OR intégrés dans les polymères de l'invention peuvent être choisis de manière non limitative parmi les structures (IV) à (XV) suivantes : 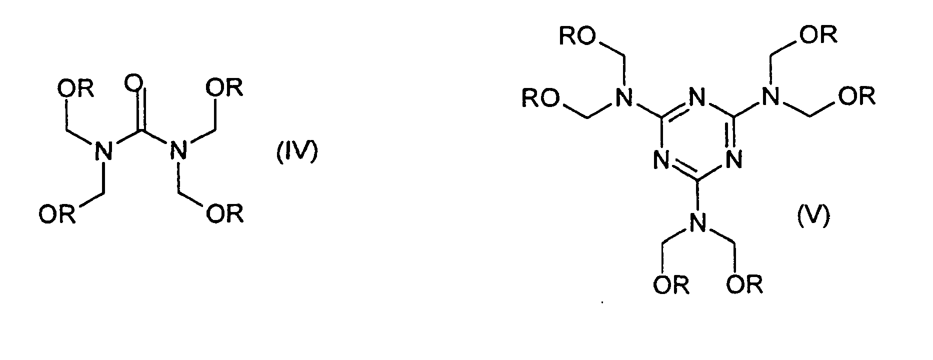 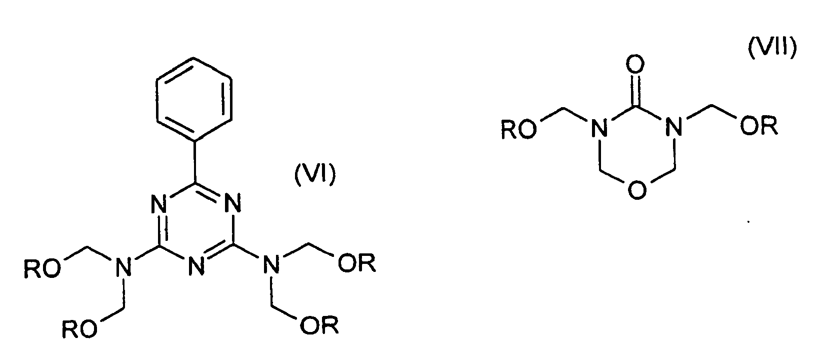 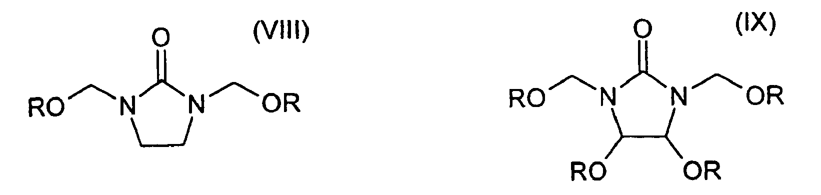 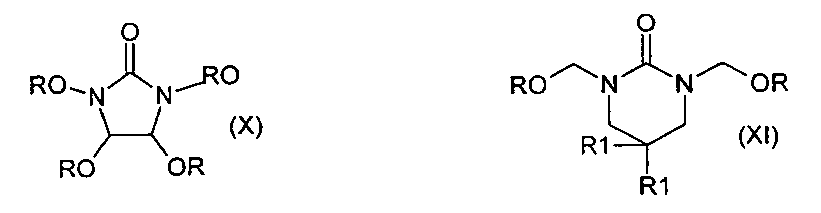 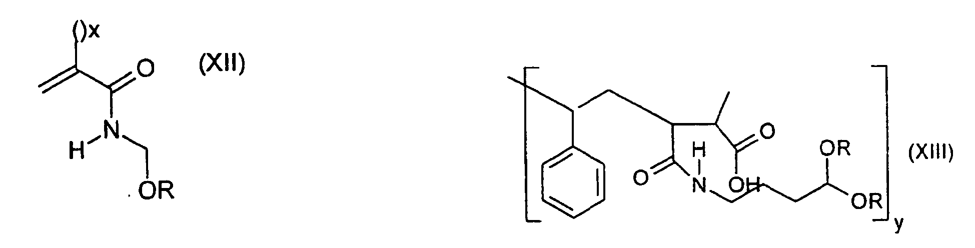 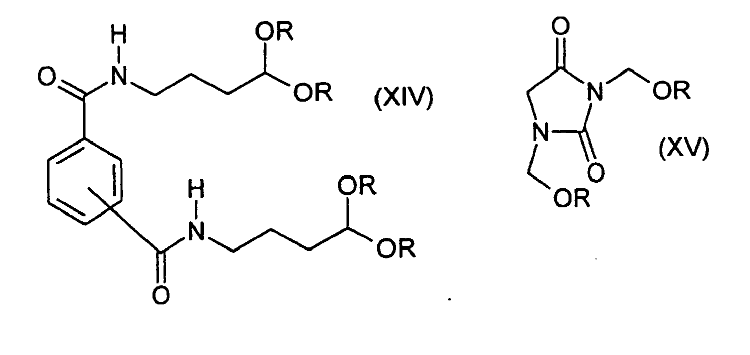 dans lesquelles :
R a la même signification que précédemment,
R1 désigne alkyle C₁C₄,
y est un nombre au moins égal à 2,
x désigne 0 ou 1.

De préférence, le ou les résidus aminoplastes porteurs de leurs groupements OR sont choisis parmi ceux de structure (XVI) suivante : 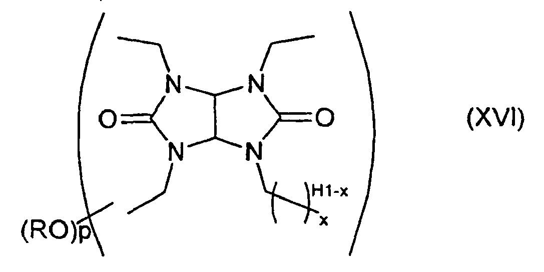 dans laquelle R, p , et x ont les mêmes significations que précédemment.

Les résidus alkylèneoxy divalents sont de préférence ceux correspondants aux diols de formule générale (XVII) suivante :

HO-(ZO)y-(Z1(Z2O)w)t-(Z'O)y'-Z3OH (XVII),

y et y' étant des nombres allant de 0 à 1000,
t et w étant des nombres allant de 0 à 10,
Z, Z', Z2 et Z3 sont des radicaux alkylène en C₂C₄ et de préférence des radicaux -CH2-CH(Z4)- et -CH2-CH(Z4)-CH2-,
Z1 étant un radical linéaire ou cyclique, ramifié ou non, aromatique ou non, comportant ou non un ou plusieurs hétéroatomes et possédant de 1 à 40 atomes de carbone,
Z4 désignant un atome d'hydrogène ou un radical alkyle en C₁C₄ ou un radical acyle en C₁C₃ étant entendu qu'au moins un des radicaux Z4 des radicaux Z, Z',
Z2 et Z3 est différent de acyle.

De préférence Z4 désigne un atome d'hydrogène ou un radical méthyle.
Encore plus préférentiellement t=0 et Z, Z' et Z3 désignent -CH2CH2-, et l'un au moins de y ou y' est différent de 0. Les composés de formule (XVII) sont alors des polyéthylèneglycols.

Les polymères aminoplaste éther de formule (1) selon l'invention sont en particulier décrits dans le brevet US 5 914 373 dont le contenu fait partie intégrante de l'invention.

Comme polymères à squelette aminoplaste-éther de formule (1), on peut en particulier citer les produits PURE-THIX L [ PEG-180/Octoxynol-40/TMMG Copolymer (Nom INCI)], PURE-THIX M [PEG-180/Laureth-50/TMMG Copolymer (Nom INCI)], et PURE-THIX HH [ Polyether-1(Nom INCI)] vendus par la société SUD-CHEMIE .

Les polymères à squelette aminoplaste-éther sont utilisés de préférence en une quantité pouvant varier d'environ 0,01 à 10% en poids du poids total de la composition de teinture prête à l'emploi. Plus préférentiellement, cette quantité varie d'environ 0,1 à 5% en poids.

Les colorants directs utilisables selon l'invention sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention , on peut citer de manière non limitative les composés suivants:
- 1,4-Diamino-2-nitrobenzène
- 1-Amino-2 nitro-4-β- hydroxyéthylaminobenzène
- 1-Amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-Amino-3-méthyl-4-β-hydroxyéthylamino-6-nitrobenzène
- 1-Amino-2-nitro-4-β-hydroxyéthylamino-5-chlorobenzène
- 1,2-Diamino-4-nitrobenzène
- 1-Amino-2-β-hydroxyéthylamino-5-nitrobenzène
- 1,2-Bis-(β-hydroxyéthylamino)-4-nitrobenzène
- 1-Amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène

Parmi les colorants directs azoïques utilisables selon l'invention on peut citer les colorants azoiques cationiques décrits dans les demandes de brevets WO 95/15144, WO-95/01772 et EP-714954 dont le contenu fait partie intégrante de l'invention.
On peut également citer parmi les colorants directs azoïques les colorants suivants, décrits dans le COLOUR INDEX INTERNATIONAL 3^{e} édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.
On peut aussi citer le 1-(4'-aminodiphénylazo)-2-méthyl-4bis-(p-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1 ,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Plus particulièrement, les compositions selon l'invention peuvent contenir en outre au moins un polymère substantif cationique ou amphotère.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 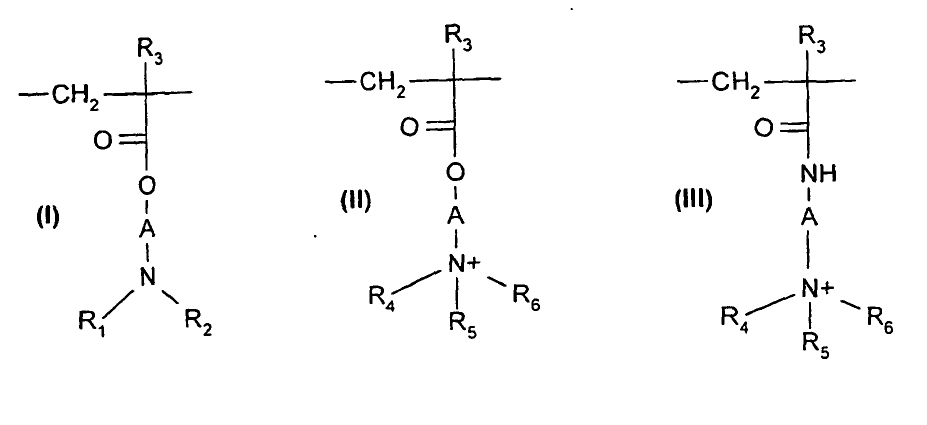 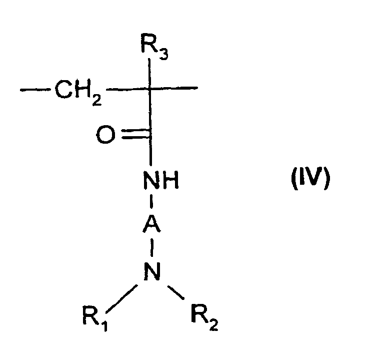 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 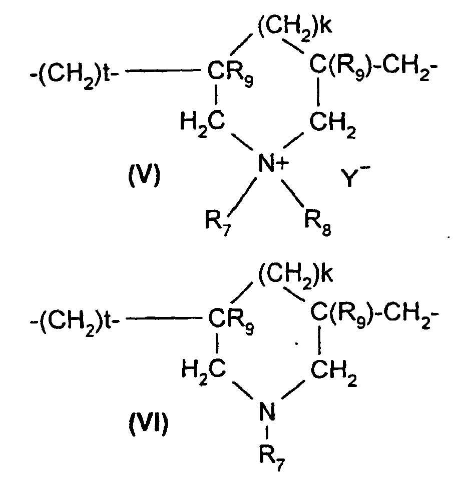 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 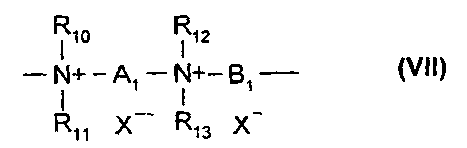 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁,
   R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.
   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 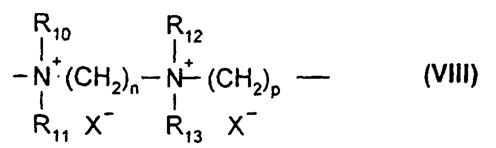 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (IX) : 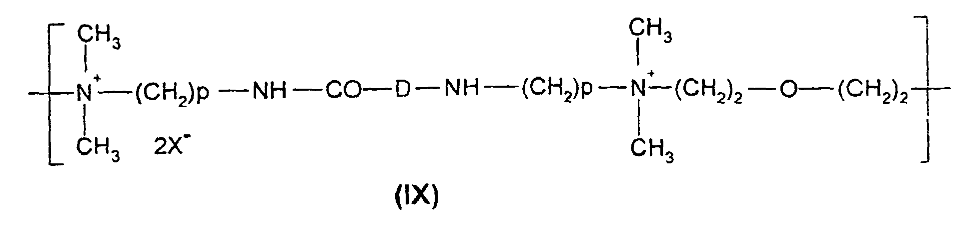 dans laquelle :
   p désigne un nombre entier variant de 1 à 6 environ,
   D peut être nul ou peut représenter un groupement ―(CH₂)ᵣ―CO― dans lequel r désigne un nombre égal à 4 ou à 7, et
   X- est un anion dérivé d'un acide minéral ou organique.

   Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.
   Parmi ces polymères, on préfère ceux de masse moléculaire mesurée par RMN du Carbone 13 inférieure à 100000, et dans la formule de laquelle p est égal à 3, et,
   a) D représente un groupement ―(CH₂)₄―CO―, X désigne un atome de chlore, la masse moléculaire mesurée par RMN du Carbone 13 ( RMN¹³C) étant d'environ 5600 ; un polymère de ce type est proposé par la société MIRANOL sous le nom de MIRAPOL-AD1,
   b) D représente un groupement ―(CH₂)₇―CO―, X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).
   Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13, ( RMN¹³C ) étant d'environ 25500.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE" dans le dictionnaire CTFA.
(14) Les polymères réticulés de sels de méthacryloyloxyalkyl(C₁-C₄) trialkyl(C₁₋C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthyiméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères de formules (W) et (U) suivantes : 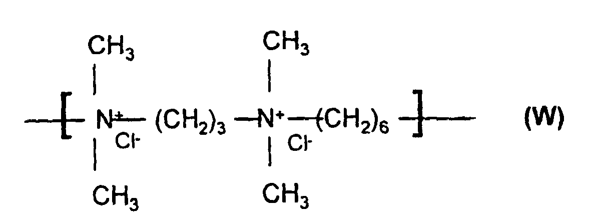 et notamment ceux dont le poids moléculaire, déterminé par chromatographie par perméation de gel, est compris entre 9500 et 9900; 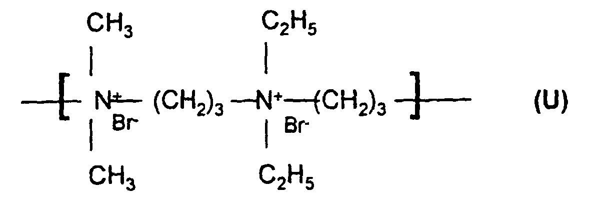 et notamment ceux dont le poids moléculaire, déterminé par chromatographie par perméation de gel, est d'environ 1200.

La concentration en polymère cationique dans les compositions selon la présente invention peut varier de 0,01 à 10% en poids par rapport au poids total de la composition, de préférence de 0,05 à 5% et plus préférentiellement encore de 0,1 à 3%.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle. On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO―R₁₉―CO―Z⁆ **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 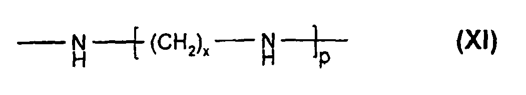 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 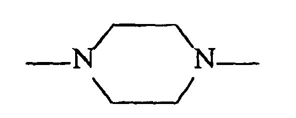
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide, chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 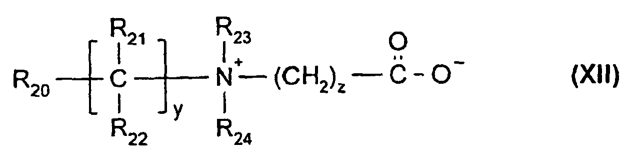 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle /diméthyl-carboxyméthylammonio-éthylméthacrylate de méthyle tel que le produit vendu sous la dénomination DIAFORMER Z301 par la société SANDOZ.
(5) les polymères dérivés du chitosane comportant des motifs monomères répondant aux formules (XIII), (XIV), (XV) suivantes : 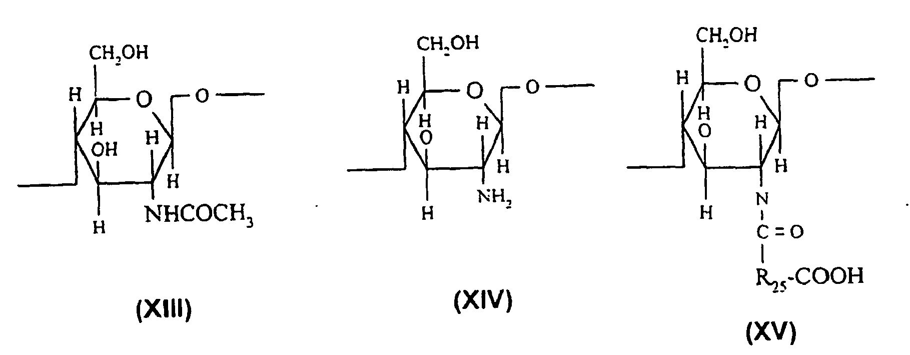 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 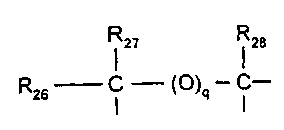 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 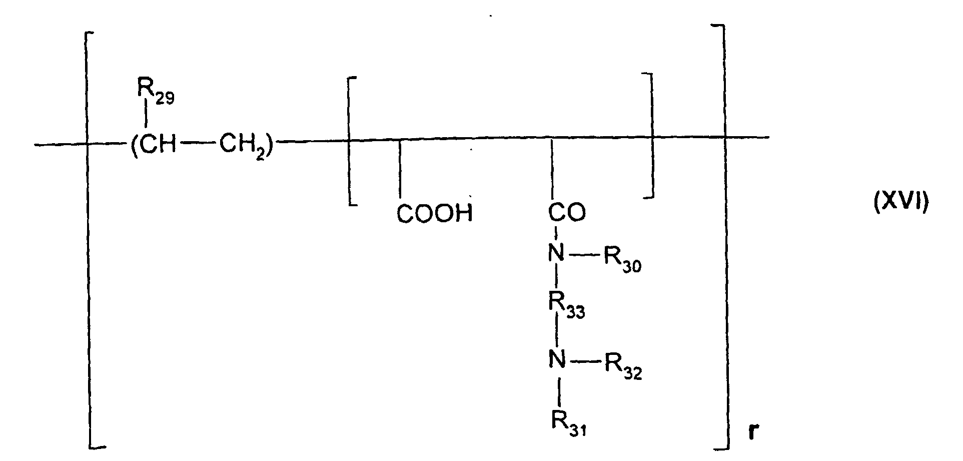 dans laquelle R₂₉ représente un atome d'hydrogène, un radical CH₃O CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 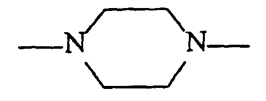 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne;
   b) les polymères de formule :

      -D-X-D-X- **(XVIII)**

      où D désigne un radical 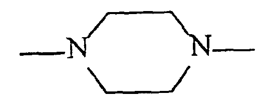 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.
Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s)

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆₋C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s):

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈₋C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₃₄ -CONHCH₂CH₂ -N(R₃₅)(R₃₆)(CH₂COO⁻)

dans laquelle : R₃₄ désigne un radical alkyle d'un acide R₃₄-COOH présent dans l'huile de coprah hydrolysée, un radical heptyle, nonyle ou undécyle, R₃₅ désigne un groupement bêta-hydroxyéthyle et R₃₆ un groupement carboxyméthyle ;
et
R₃₄'-CONHCH₂CH₂-N(B)(C)
dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R₃₄' désigne un radical alkyle d'un acide R₃₇ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement palyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition prête à l'emploi selon l'invention peuvent varier de 0,01 à 40% et de préférence de 0,1 à 30% du poids total de la composition.

Les compositions selon l'invention peuvent également contenir d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition peut encore contenir une quantité efficace d'autres agents, par ailleurs antérieurement connus en coloration directe, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des agents antioxydants, etc....

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi avec agent oxydant, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi [composition sans oxydant prête à l'emploi ou composition résultant du mélange de la composition tinctoriale A1 et de la composition oxydante B1], est généralement compris entre les valeurs 2 et 12. Il est de préférence compris entre 3 et 11, et peut être ajusté à la valeur désirée au moyen d'agents acidifiants ou alcalinisants bien connus de l'état de la technique en teinture des fibres kératiniques.
Plus préférentiellement lorsque la composition contient un agent oxydant pour l'éclaircissement des fibres, le pH du mélange prêt à l'emploi est supérieur à 7 et encore plus préférentiellement supérieur à 8.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 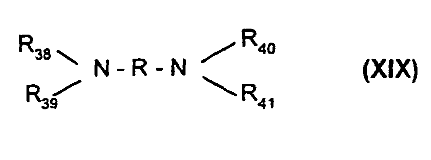 dans laquelle R est un reste propylène éventuellement substitué par un groupement hydroxyle ou un radical alkyle en C₁-C₄ R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition sans oxydant prête à l'emploi A, ou la composition réalisée extemporanément au moment de l'emploi à partir des compositions A1 et B1 décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Une variante de ce procédé consiste à prendre une composition A' contenant au moins un colorant direct mais sans polymère à squelette aminoplaste-éther, une composition A" contenant au moins un polymère à squelette aminoplaste-éther et à mélanger au moment de l'emploi ces compositions A', A" avec la composition B oxydante, puis à appliquer et laisser agir le mélange comme précédemment.

Des exemples concrets illustrant l'invention sont indiqués ci-après, sans pour autant présenter un caractère limitatif.

### EXEMPLE 1 :

On a préparé les compositions suivantes : (exprimées en grammes)

**Composition oxydante :**

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée ....qsp ...... | 100 |

**Composition colorante :**

| | |
|---|---|
| alcools gras oxyéthylénés..... | 32,5 |
| Acide oléique...................... | 2 |
| Alcool oléique..................... | 1,8 |
| Amide gras........................ | 4 |
| Glycérine.......................... | 3 |
| Polymère cationique de formule (W) en solution à 60% dans l'eau................... | 2 |
| Merquat280...................... | 2 |
| Agent séquestrant............... | qs |
| Réducteur......................... | qs |
| Ammoniaque (20% NH3)...... | 8 |
| Diamino-1,4-nitro-2-benzène | 0,6 |
| PURE-THIX-HH (INCI=POLYETHER-1) ........ | 0,3 MA* |
| Eau........................ qsp..... | 100 |

| | |
|---|---|
| MA* = Matière Acitve | |

La composition colorante a été mélangée, au moment de l'emploi, dans un bol en plastique et pendant 2 minutes, à la composition oxydante donnée ci-dessus, à raison de 1 partie de composition colorante pour 1,5 parties de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées et démélées.
Les cheveux ont été teints dans une nuance rouge puissante.

### EXEMPLE 2

On a préparé la composition suivante :
(exprimées en grammes)

| | |
|---|---|
| alcools gras oxyéthylénés..... | 32,5 |
| Acide oléique | 2 |
| Alcool oléique ..................... | 1,8 |
| Amide gras........................ | 4 |
| Glycérine.......................... | 3 |
| Basic Red 76 | 0,4 |
| PURE-THIX-HH (INCI=POLYETHER-1) ...... | 0,3 MA* |
| Amino-2-méthyl-2-propanol-1 qs.................................... | pH 8,5 |
| Eau........................ qsp..... | 100 |

| | |
|---|---|
| MA* = Matière Active | |

Cette composition a été appliquée trente minutes sur des cheveux gris à 90% de blancs. Après rinçage et séchage les cheveux ont été colorés en rouge intense.

## Revendications

1. Composition de teinture directe pour fibres kératiniques, en particulier pour fibres kératiniques humaines telles que les cheveux comprenant dans un milieu approprié pour la teinture, au moins un colorant direct, **caractérisée par le fait qu'**elle contient en outre au moins un polymère épaississant à squelette aminoplaste-éther.

2. Composition selon la revendication 1, **caractérisée par le fait que** le ou les polymères aminoplaste-éther sont choisis parmi ceux contenant au moins un motif de structure (I) suivante : 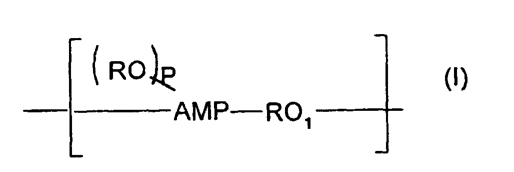 dans laquelle :
- AMP est un résidu aminoplaste avec unités alkylènes,
- R désigne un atome d'hydrogène, un radical alkyl C₁C₄ ou un radical acyle C₁C₄,
- RO₁ est un résidu alkylèneoxy divalent,
- p désigne un nombre entier positif,
le ou les groupements OR étant liés aux unités alkylènes du résidu AMP.

3. Composition selon les revendications 1 ou 2, **caractérisée par le fait que** le ou les polymères à squelette aminoplaste-éther sont choisis parmi ceux contenant au moins un motif de structure (II) suivante : 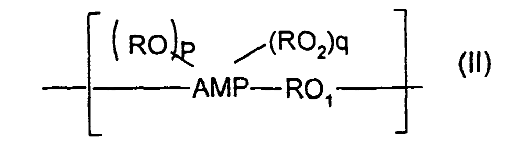 dans laquelle :
- AMP, R, RO₁ et p ont la même signification que dans la revendication 2,
- RO₂ est un groupe hydrophobe différent de RO lié à AMP via un hétéroatome et comprenant au moins deux atomes de carbone, et,
- q est un nombre entier positif.

4. Composition selon la revendication 3, **caractérisée par le fait que** le ou les polymères aminoplaste-éther sont choisis parmi les polymères de structure (III) ou (IIIbis) suivantes : 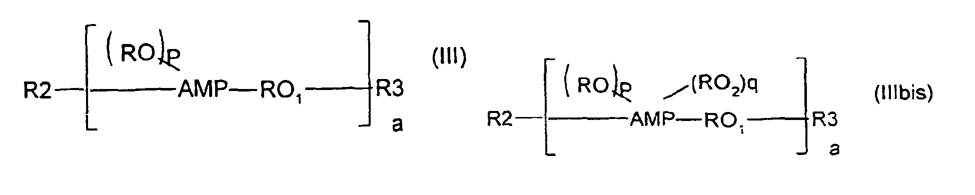 dans lesquelles :
AMP, R, RO₁, RO₂, p et q ont la même signification que dans les revendications 2 et 3,
R2 ou R3, identiques ou différents représentent un groupe terminal pouvant désigner un atome d'hydrogène, un groupement RO₁H, un groupement RO₂H un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant un nombre plus grand que 1 et de préférence plus grand que 2.

5. Composition selon l'une quelconque des revendications 2 à 4, **caractérisée par le fait que** les résidus aminoplastes porteurs de leurs groupements OR sont choisis parmi ceux de structures (IV) à (XV) suivantes : 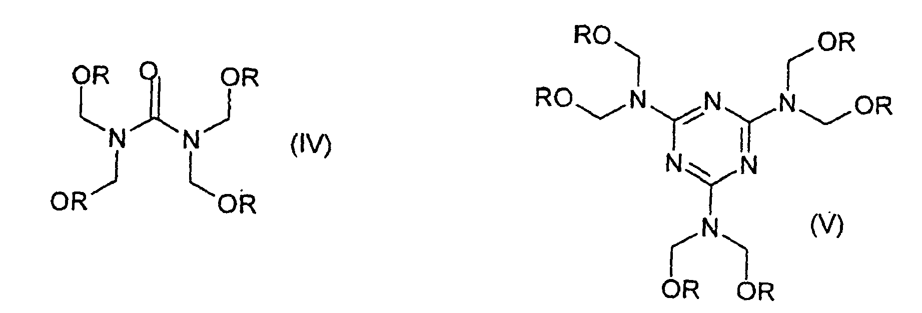 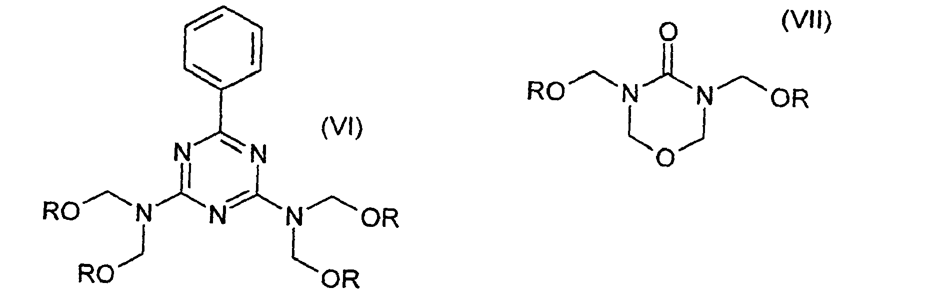 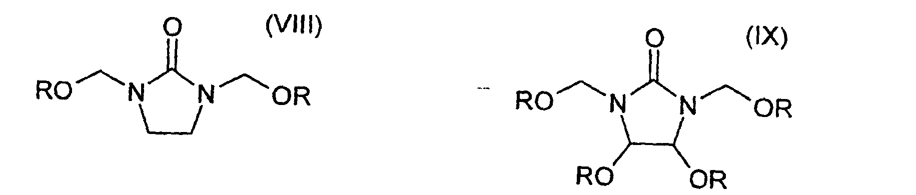 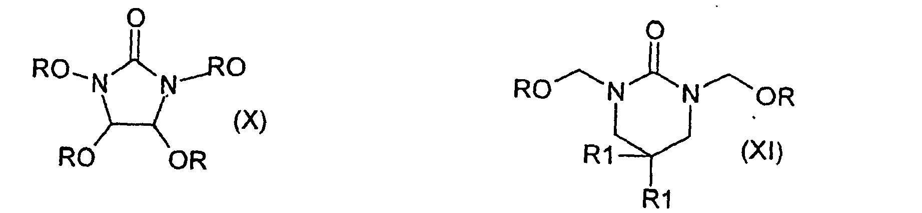 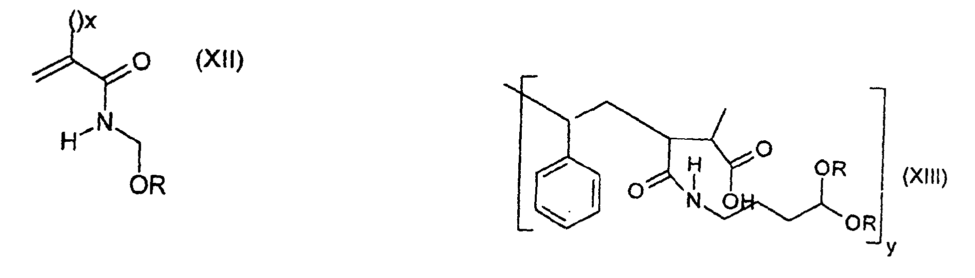 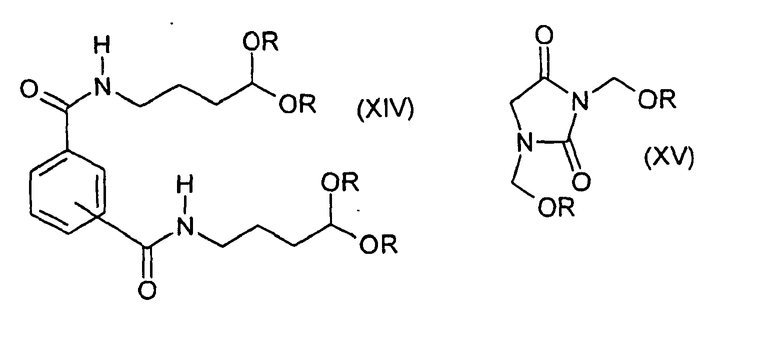
R ayant la même signification que dans la revendication 2,
R1 désigne alkyle C₁C₄,
y est un nombre au moins égal à 2,
x désigne 0 ou 1.

6. Composition selon l'une quelconque des revendications 2 à 4, **caractérisée par le fait que** les résidus aminoplastes porteurs de leurs groupements OR sont choisis parmi ceux de structure (XVI) suivante : 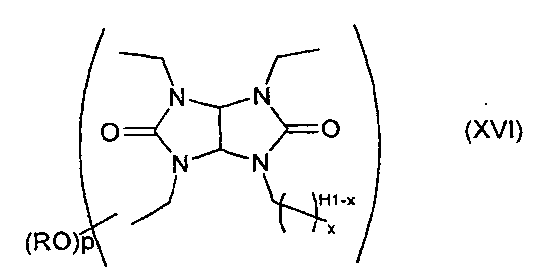 dans laquelle R, p , et x ont les mêmes significations que dans les revendications 2 et 5.

7. Composition selon l'une queconque des revendications 2 à 6, **caractérisée par le fait que** les résidus alkylèneoxy sont ceux correspondants aux diols de formule générale (XVII) suivante :
HO-(ZO)y-(Z1(Z2O)w)t-(Z'O)y'-Z3OH (XVII),
y et y' étant des nombres allant de 0 à 1000,
t et w étant des nombres allant de 0 à 10,
Z, Z', Z2 et Z3 sont des radicaux alkylène en C₂C₄ et de préférence des radicaux -CH2-CH(Z4)- et -CH2-CH(Z4)-CH2-,
Z1 étant un radical linéaire ou cyclique, ramifié ou non, aromatique ou non, comportant ou non un ou plusieurs hétéroatomes et possédant de 1 à 40 atomes de carbone,
Z4 désignant un atome d'hydrogène ou un radical alkyle en C₁C₄ ou un radical acyle en C₁C₃ étant entendu qu'au moins un des radicaux Z4 des radicaux Z, Z',
Z2 et Z3 est différent de acyle.

8. Composition selon la revendication 7, **caractérisée par le fait que** le radical Z4 désigne un atome d' hydrogène ou un radical méthyle.

9. Composition selon les revendications 7 et 8, **caractérisée par le fait que** t=0 et que Z, Z' et Z3 désignent -CH2CH2- , l'un au moins de y ou y' étant différent de 0.

10. Composition selon l'une quelconque des revendications précédentes, **caractérisée par le fait que** les polymères à squelette aminoplaste-éther sont choisi parmi les produits suivants :
PEG-180/Octoxynol-40/TMMG Copolymer,
PEG-180/Laureth-50/TMMG Copolymer,
Polyether-1.

11. Composition selon l'une quelconque des revendications 1 à 10, **caractérisée par le fait que** les polymères à squelette aminoplaste-éther sont utilisés en une quantité pouvant varier de 0,01 à 10% en poids du poids total de la composition.

12. Composition selon la revendication 11, **caractérisée par le fait que** les polymères à squelette aminoplaste-éther sont utilisés en une quantité pouvant varier de 0,1 à 5% en poids du poids total de la composition.

13. Composition selon l'une quelconque des revendications précédentes, **caractérisée par le fait que** le colorant direct est choisi parmi les colorants directs nitrés benzéniques neutres, acides ou cationiques, les colorants directs azoïques neutres acides ou cationiques, les colorants directs quinoniques et en particulier anthraquinoniques, neutres, acides ou cationiques, les colorants directs aziniques, les colorants directs triarylméthaniques, les colorants directs indoaminiques et les colorants directs naturels.

14. Composition selon l'une quelconque des revendications précédentes, **caractérisée par le fait que** le ou les colorants directs sont présents dans des concentrations allant de 0,001 à 20% et de préférence de 0,005 à 10% en poids par rapport au poids total de la composition.

15. Composition selon l'une quelconque des revendications précédentes, **caractérisée par le fait qu'**elle contient en outre au moins un polymère cationique ou amphotère.

16. Composition selon la revendication 15, **caractérisée par le fait que** le polymère cationique est un polymère de polyammonium quaternaire constitué de motifs récurrents répondant à la formule (W) suivante :

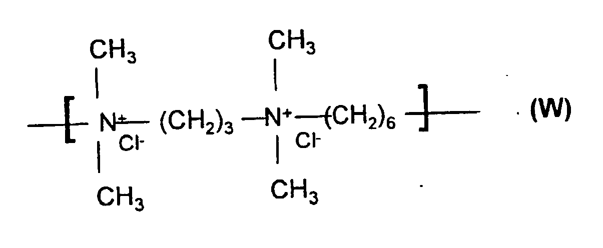

17. Composition selon la revendication 15, **caractérisée par le fait que** le polymère cationique est un polymère de polyammonium quaternaire constitué de motifs récurrents répondant à la formule (U) suivante :

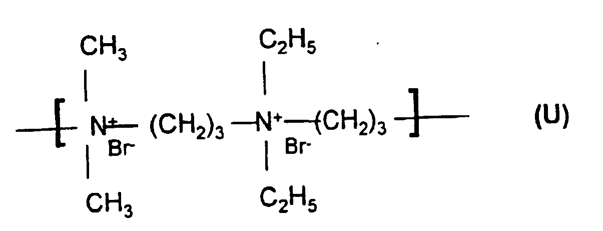

18. Composition selon la revendication 15, **caractérisée par le fait que** le polymère amphotère est un copolymère comprenant au moins comme monomères de l'acide acrylique et un sel de diméthyldiallylammonium.

19. Composition selon l'une quelconque des revendications 15 à 18, **caractérisée par le fait que** le ou les polymères cationiques ou amphotères représentent de 0,01 % à 10 %, de préférence de 0,05 % à 5 %, et encore plus préférentiellement de 0,1 % à 3 % en poids, du poids total de la composition.

20. Composition selon l'une quelconque des revendications précédentes, **caractérisée par le fait qu'**elle contient au moins un tensioactif choisi parmi les tensioactifs anioniques, cationiques, non ioniques ou amphotères.

21. Composition selon la revendication 20, **caractérisée par le fait que** les tensioactifs représentent 0,01 à 40% et de préférence 0,1 à 30% en poids du poids total de la composition.

22. Composition selon l'une quelconque des revendications précédentes, **caractérisee par le fait qu'**elle contient au moins un agent épaississant additionnel.

23. Composition selon la revendication 22 , **caractérisée par le fait que** l'agent épaississant additionnel est un dérivé de cellulose, un dérivé de guar, une gomme d'origine microbienne, un épaississant synthétique.

24. Composition selon la revendication 23, **caractérisée par le fait que** le ou les épaississants additionnels représentent 0,01 à 10% en poids du poids total de la composition prête à l'emploi.

25. Composition selon l'une quelconque des revendications précédentes, **caractérisée par le fait qu'**elle possède un pH allant de 2 à 12.

26. Composition prête à l'emploi selon l'une quelconque des revendications précédentes, **caractérisée par le fait qu'**elle contient en outre un agent oxydant.

27. Composition selon la revendication 26, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

28. Composition selon la revendication 27, **caractérisée par le fait que** l'agent oxydant est le peroxyde d'hydrogène.

29. Composition selon la revendication 28, **caractérisée par le fait qu'**il s'agit d'une solution d'eau oxygénée dont le titre varie de 1 à 40 volumes.

30. Composition selon l'une quelconque des revendications 26 à 29, **caractérisée par le fait qu'**elle possède un pH supérieur à 7 et de préférence supérieur à 8.

31. Procédé de teinture directe des fibres kératiniques et en particulier des fibres kératiniques humaines telles que les cheveux, **caractérisé par le fait qu'**il consiste à appliquer sur les fibres une composition de teinture A telle que définie à l'une quelconque des revendications 1 à 25.

32. Procédé de teinture directe éclaircissante des fibres kératiniques et en particulier des fibres kératiniques humaines telles que les cheveux, **caractérisé par le fait qu'**il consiste à appliquer sur les fibres une composition résultant du mélange extemporané d'une composition A1 contenant, dans un milieu approprié pour la teinture, au moins un colorant direct, et d'une composition oxydante B1 contenant un agent oxydant, la composition A1 et/ou la composition B1 contenant au moins un polymère à squelette aminoplaste-éther tel que défini à l'une quelconque des revendications 1 à 12.

33. Procédé selon les revendications 31 ou 32, **caractérisé par le fait qu'**il consiste à appliquer sur les fibres kératiniques sèches ou humides, la composition prête à l'emploi A ou celle réalisée extemporanément au moment de l'emploi à partir des compositions A1 et B1, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

34. Procédé de teinture directe éclaircissante des fibres kératiniques et en particulier des fibres kératiniques humaines telles que les cheveux, **caractérisé par le fait qu'**il consiste à appliquer sur les fibres kératiniques sèches ou humides, la composition prête à l'emploi réalisée extemporanément au moment de l'emploi à partir d'une composition A' comprenant au moins un colorant direct, mais sans polymère à squelette aminoplaste-éther défini aux revendications 1-12, une autre composition A" contenant au moins un polymère à squelette aminoplaste-éther défini selon les revendications 1 à 12, et une composition oxydante B, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

35. Procédé de teinture selon l'une quelconque des revendications 31-34, **caractérisé par le fait que** la composition A ou A1 et/ou la composition B1 renferment au moins un polymère cationique ou amphotère et au moins un tensioactif.

36. Dispositif à deux compartiments ou " Kit " pour la teinture directe des fibres kératiniques et en particulier des fibres kératiniques humaines telles que les cheveux, **caractérisé par le fait qu'**un compartiment renferme une composition contenant au moins un colorant direct, et un autre compartiment renferme une composition comprenant au moins un polymère à squelette aminoplaste-éther tel que défini à l'une quelconque des revendications 1 à 12.

37. Dispositifs à deux compartiments ou "Kits" pour la teinture directe éclaircissante des fibres kératiniques et en particulier des fibres kératiniques humaines telles que les cheveux, **caractérisé par le fait qu'**un compartiment renferme une composition A1 contenant, dans un milieu approprié pour la teinture, au moins un colorant direct, et un autre compartiment renferme une composition B1 contenant un agent oxydant, au moins un polymère à squelette aminoplaste-éther étant présent dans la composition A1 ou dans la composition B1, ou dans chacune des compositions A1 et B1.

## Patentansprüche

1. Zusammensetzung für die Direktfärbung von Keratinfasern und insbesondere menschlichen Keratinfasern, wie Haaren, die in einem zum Färben geeigneten Medium mindestens einen Direktfarbstoff enthält, **dadurch gekennzeichnet, dass** sie ferner mindestens ein verdickendes Polymer mit Aminoplast-Ether-Grundstruktur enthält.

2. Zusammensetzung nach Anspruch 1, **dadurch gekennzeichnet, dass** das oder die Aminoplast-Ether-Polymer(e) unter den Polymeren ausgewählt sind, die mindestens eine Einheit der folgenden Struktur (I) enthalten: 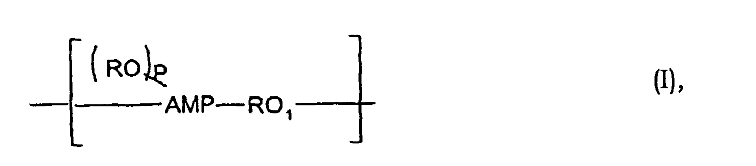 worin bedeuten:
- AMP einen Aminoplastrest mit Alkyleneinheiten,
- R ein Wasserstoffatom, eine C₁₋₄-Alkylgruppe oder eine C₁₋₄₋Acylgruppe,
- RO₁ einen zweiwertigen Alkylenoxyrest,
- p eine positive ganze Zahl,
wobei die Gruppe(n) OR an die Alkyleneinheiten des AMP-Rests gebunden sind

3. Zusammensetzung nach Anspruch 1 oder 2, **dadurch gekennzeichnet, dass** das oder die Polymere(e) mit Aminoplast-Ether-Grundgerüst unter den Polymeren ausgewählt sind, die mindestens eine Einheit der folgenden Struktur (II) enthalten: 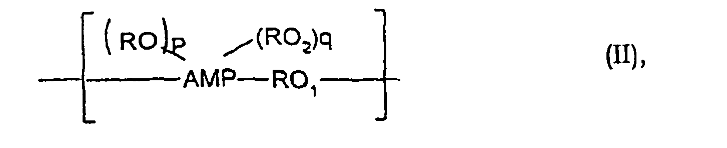 worin bedeuten:
- AMP, R, RO₁ und p die in Anspruch 2 angegebenen Bedeutungen,
- RO₂ eine hydrophobe Gruppe, die von RO verschieden ist und über ein Heteroatom an AMP gebunden ist und mindestens zwei Kohlenstoffatome aufweist, und
- q eine positive ganze Zahl.

4. Zusammensetzung nach Anspruch 3, **dadurch gekennzeichnet, dass** das oder die Aminoplast-Ether-Polymer(e) unter den Polymeren der folgenden Strukturen (III) oder (IIIbis) ausgewählt sind: 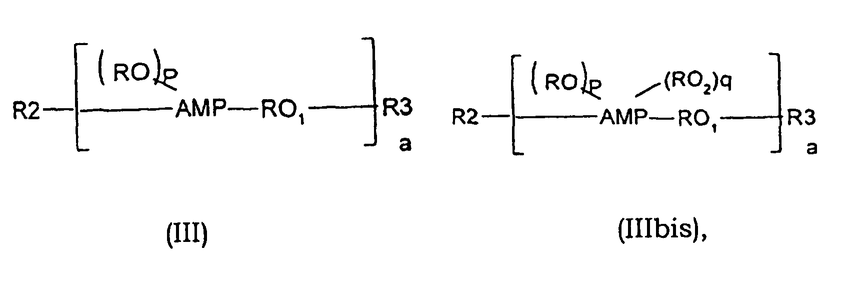 worin bedeuten:
AMP, R, RO₁, RO₂, p und q die in den Ansprüchen 2 und 3 angegebenen Bedeutungen,
R2 oder R3, die gleich oder verschieden sind, eine endständige Gruppe, die ein Wasserstoffatom, eine Gruppe RO₁H, eine Gruppe RO₂H, eine Gruppe AMP(OR)p oder beliebige monofunktionelle Gruppen bedeuten kann, wie Alkyl, Cycloalkyl, Aryl, Aralkyl, Alkylaryl, Alkyloxyalkyl, Aryloxyalkyl, Cycloalkoxyalkyl,
wobei a eine Zahl > 1 und vorzugsweise >2 bedeutet.

5. Zusammensetzung nach einem der Ansprüche 2 bis 4, **dadurch gekennzeichnet, dass** die Aminoplastreste, die Gruppen OR aufweisen, unter den Strukturen der folgenden Formeln (IV) bis (XV) ausgewählt sind: 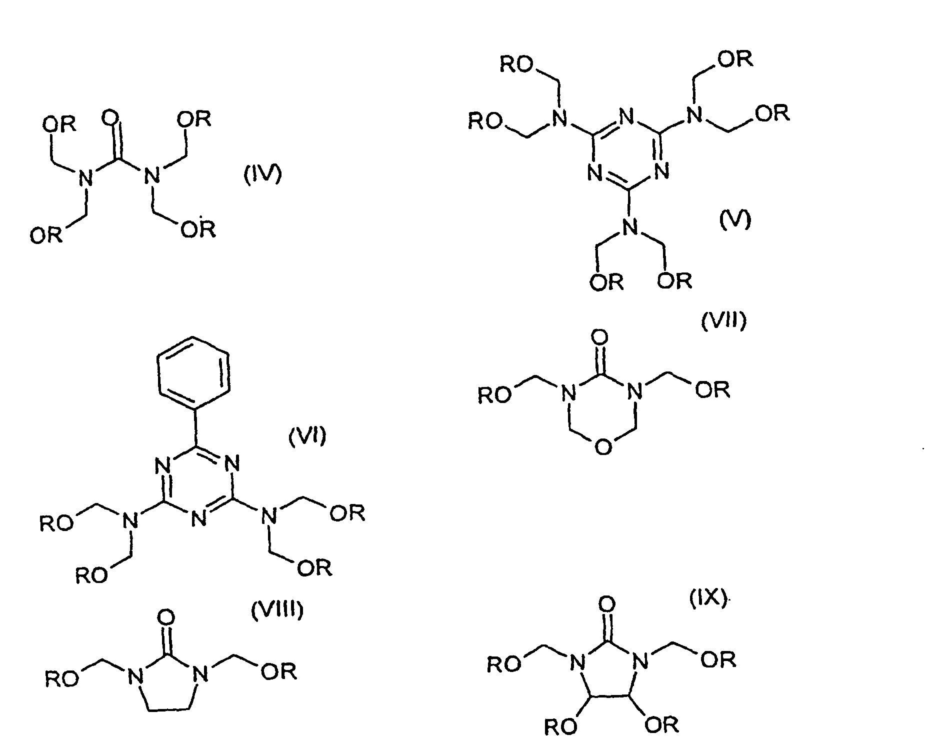 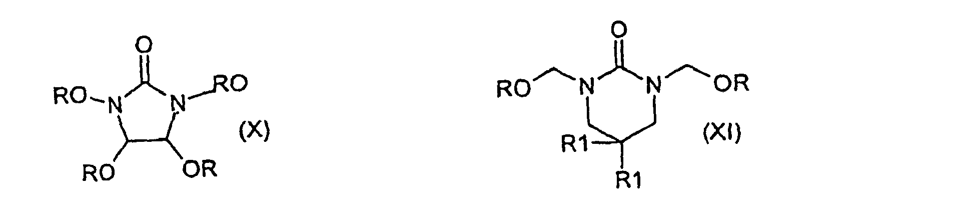 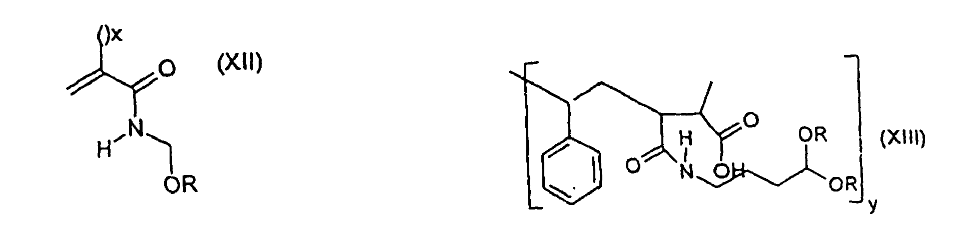 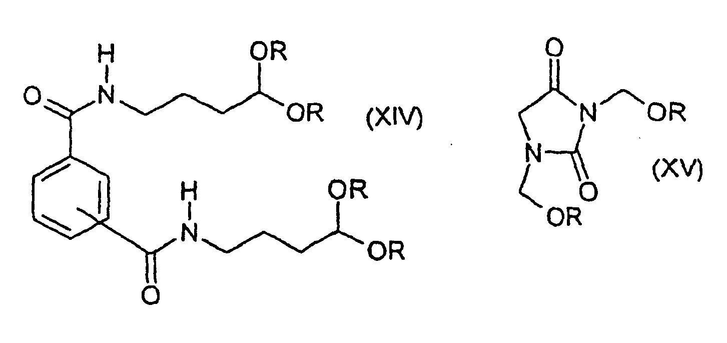 wobei R die in Anspruch 2 angegebene Bedeutung aufweist,
R1 C₁₋₄-Alkyl bedeutet,
y eine Zahl von mindestens 2 ist,
x 0 oder 1 bedeutet.

6. Zusammensetzung nach einem der Ansprüche 2 bis 4, **dadurch gekennzeichnet, dass** die Aminoplastreste, die OR-Gruppen tragen, unter den Resten der folgenden Struktur (XVI) ausgewählt sind: 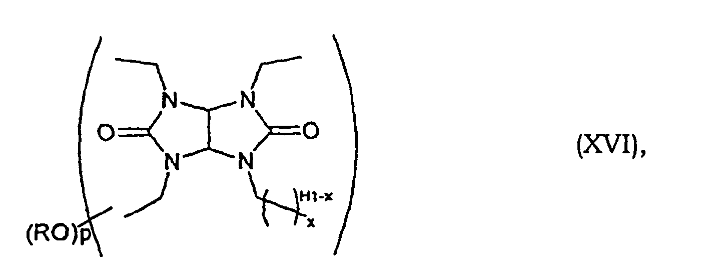 worin R, p und x die in den Ansprüchen 2 und 5 angegebenen Bedeutungen aufweisen.

7. Zusammensetzung nach einem der Ansprüche 2 bis 6, **dadurch gekennzeichnet, dass** die Alkylenoxyreste Gruppen sind, die Diolen der allgemeinen Formel (XVII) entsprechen:
HO-(ZO)y-(Z1(Z2O)w)t-(Z'O)y'-Z3OH (XVII),
wobei y und y' Zahlen von 0 bis 1 000 bedeuten,
1 und w Zahlen von 0 bis 10 bedeuten,
Z, Z', Z2 und Z3 C₂₋₄-Alkylengruppen und vorzugsweise die Gruppen
-CH2-CH(Z4-) und -CH2-CH(Z4)-CH2- bedeuten,
wobei Z1 eine geradkettige oder cyclische, verzweigte oder nicht verzweigte, aromatische oder nicht aromatische Gruppe ist, die gegebenenfalls ein oder mehrere Heteroatome enthält und 1 bis 40 Kohlenstoffatome aufweist,
Z4 ein Wasserstoffatom, C₁₋₄-Alkyl oder C₁₋₃-Acyl bedeutet, mit der Maßgabe, dass mindestens eine Gruppe Z4 der Gruppen Z,
Z', Z2 und Z3 von Acyl verschieden ist.

8. Zusammensetzung nach Anspruch 7, **dadurch gekennzeichnet, dass** die Gruppe Z4 ein Wasserstoffatom oder die Methylgruppe bedeutet.

9. Zusammensetzung nach Anspruch 7 oder 8, **dadurch gekennzeichnet, dass** t=0 und Z, Z' und Z3 -CH2CH2- bedeuten, wobei mindestens ein Wert y oder y' von 0 verschieden ist.

10. Zusammensetzung nach einem der vorhergehenden Ansprüche, **dadurch gekennzeichnet, dass** die Polymere mit Aminoplast-Ether-Grundgerüst unter den folgenden Produkten ausgewählt sind:
PEG-180/Octoxynol-40/TMMG Copolymer,
PEG-180/Laureth-50/TMMG Copolymer,
Polyether-1.

11. Zusammensetzung nach einem der Ansprüche 1 bis 10, **dadurch gekennzeichnet, dass** die Polymere mit Aminoplast-Ether-Grundgerüst in einer Menge verwendet werden, die im Bereich von 0,01 bis 10 Gew.-% des Gesamtgewichts der Zusammensetzung liegen kann.

12. Zusammensetzung nach Anspruch 11, **dadurch gekennzeichnet, dass** die Polymere mit Aminoplast-Ether-Grundgerüst in einer Menge verwendet werden, die im Bereich von 0,1 bis 5 Gew.-% des Gesamtgewichts der Zusammensetzung liegen kann.

13. Zusammensetzung nach einem der vorhergehenden Ansprüche, **dadurch gekennzeichnet, dass** der Direktfarbstoff unter den neutralen, sauren oder kationischen direktziehenden Benzolfarbstoffen, neutralen, sauren oder kationischen direktziehenden Azofarbstoffen, neutralen, sauren oder kationischen direktziehenden Chinonfarbstoffen, insbesondere AnthrachinonFarbstoffen, direktziehenden Azinfarbstoffen, direktziehenden Triarylmethanfarbstoffen, direktziehenden Indoaminfarbstoffen und direktziehenden natürlichen Farbstoffen ausgewählt ist.

14. Zusammensetzung nach einem der voerhergehenden Ansprüche, **dadurch gekennzeichnet, dass** der oder die Direktfarbstoff(e) in Konzentrationen von 0,001 bis 20 % und vorzusweise 0,005 bis 10 Gew.-%, bezogen auf das Gesamtgewicht der Zusammensetzung, enthalten sind.

15. Zusammensetzung nach einem der vorhergehenden Ansprüche, **dadurch gekennzeichnet, dass** sie ferner mindestens ein kationisches oder amphoteres Polymer enthält.

16. Zusammensetzung nach Anspruch 15, **dadurch gekennzeichnet, dass** das kationische Polymer ein quartären Polyammoniumpolymer ist, das aus wiederkehrenden Einheiten der folgenden Formel (W) besteht:

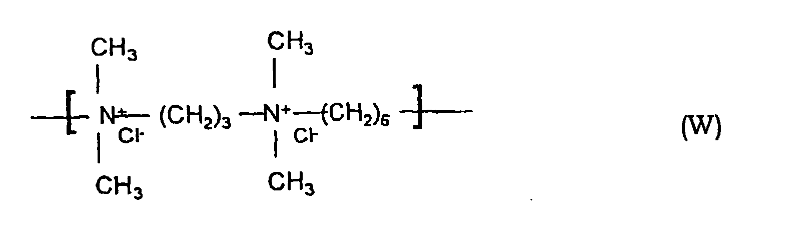

17. Zusammensetzung nach Anspruch 15, **dadurch gekennzeichnet, dass** das kationische Polymer ein quartäres Polyammoniumpolymer ist, das aus wiederkehrenden Einheiten der folgenden Formel (U) besteht:

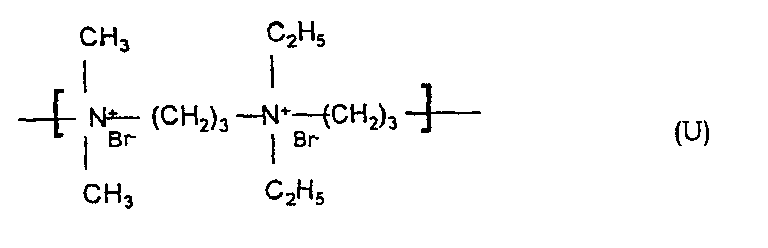

18. Zusammensetzung nach Anspruch 15, **dadurch gekennzeichnet, dass** das amphotere Polymer ein Copolymer ist, das als Monomere zumindest Acrylsäure und ein Dimethyldiallylammoniumsalz enthält.

19. Zusammensetzung nach einem der Ansprüche 15 bis 18, **dadurch gekennzeichnet, dass** das oder die kationische(n) oder amphotere(n) Polymer(e) 0,01 bis 10 %, vorzugsweise 0,05 bis 5 % und noch bevorzugter 0,1 bis 3 Gew.-% des Gesamtgewichts der Zusammensetzung ausmachen.

20. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

21. Zusammensetzung nach Anspruch 20, **dadurch gekennzeichnet, dass** die grenzflächenaktiven Stoffe 0,01 bis 40 % und vorzugsweise 0,1 bis 30 Gew.-% des Gesamtgewichts der Zusammensetzung ausmachen.

22. Zusammensetzung nach einem der vorhergehenden Ansprüche, **dadurch gekennzeichnet, dass** sie mindestens ein zusätzliches Verdickungsmittel enthält.

23. Zusammensetzung nach Anspruch 22, **dadurch gekennzeichnet, dass** das zusätzliche Verdickungsmittel ein Cellulosederivat, Guarderivat, Gummi mikrobieller Herkunft oder synthetisches Verdickungsmittel ist.

24. Zusammensetzung nach Anspruch 23, **dadurch gekennzeichnet, dass** das oder die ergänzende(n) Verdickungsmittel 0,01 bis 10 Gew.-% des Gesamtgewichts der gebrauchsfertigen Zusammensetzung ausmachen.

25. Zusammensetzung nach einem der vorhergehenden Ansprüche, **dadurch gekennzeichnet, dass** sie einen pH-Wert von 2 bis 12 aufweist.

26. Gebrauchsfertige Zusammensetzung nach einem der vorhergehenden Ansprüche, **dadurch gekennzeichnet, dass** sie ferner ein Oxidationsmittel enthält.

27. Zusammensetzung nach Anspruch 26, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls mit ihrem jeweiligen Donor oder Cofaktor ausgewählt ist.

28. Zusammensetzung nach Anspruch 27, **dadurch gekennzeichnet, dass** das Oxidationsmittel Wasserstoffperoxid ist.

29. Zusammensetzung nach Anspruch 28, **dadurch gekennzeichnet, dass** es sich um eine Wasserstoffperoxidlösung mit einem Titer von 1 bis 40 Volumina handelt.

30. Zusammensetzung nach einem der Ansprüche 26 bis 29, **dadurch gekennzeichnet, dass** sie einen pH-Wert über 7 und vorzugsweise über 8 aufweist.

31. Verfahren zur Direktfärbung von Keratinfasern und insbesondere menschlichen Keratinfasern wie Haaren, **dadurch gekennzeichnet, dass** es darin besteht, auf die Fasern eine Farbmittelzusammensetzung A nach einem der Ansprüche 1 bis 25 aufzutragen.

32. Verfahren für die aufhellende Direktfärbung von Keratinfasern und insbesondere menschlichen Keratinfasern wie Haaren, **dadurch gekennzeichnet, dass** es darin besteht, auf die Fasern eine Zusammensetzung aufzutragen, die durch bedarfsgemäßes Mischen einer Zusammensetzung A1, die in einem zum Färben geeigneten Medium mindestens einen Direktfarbstoff enthält, und einer oxidierenden Zusammensetzung B1 hergestellt wird, die ein Oxidationsmittel enthält, wobei die Zusammensetzung A1 und/oder die Zusammensetzung B1 mindestens ein Polymer mit einem Aminoplast-Ether-Grundgerüst nach einem der Ansprüche 1 bis 12 enthält.

33. Verfahren nach den Ansprüchen 31 oder 32, **dadurch gekennzeichnet, dass** es darin besteht, auf die trockenen oder feuchten Keratinfasern die gebrauchsfertige Zusammensetzung A oder die bedarfsgemäß bei der Anwendung aus den Zusammensetzungen A1 und B1 hergestellte Zusammensetzung aufzutragen, während einer Einwirkzeit von etwa 1 bis 60 min und vorzugsweise 10 bis 45 min einwirken zu lassen, die Fasern zu spülen, gegebenenfalls mit Haarwaschmittel zu waschen, nochmals zu spülen und zu trocknen.

34. Verfahren zur aufhellenden Direktfärbung von Keratinfasern und insbesondere menschlichen Keratinfasern wie Haaren, **dadurch gekennzeichnet, dass** es darin besteht, auf die trockenen oder feuchten Keratinfasern eine bedarfsgemäß bei der Anwendung aus einer Zusammensetzung A', die mindestens einen Direktfarbstoff, jedoch kein Polymer mit einem Aminoplast-Ether-Grundgerüst nach den Ansprüchen 1 und 12 enthält, einer Zusammensetzung A", die mindestens ein Polymer mit Aminoplast-Ether-Grundgerüst nach einem der Ansprüche 1 bis 12 enthält, und einer oxidierenden Zusammensetzung B hergestellte Zusammensetzung aufzutragen, während einer Einwirkzeit von etwa 1 bis 60 min und vorzugsweise 10 bis 45 min einwirken zu lassen, die Fasern zu spülen, gegebenenfalls mit Haarwaschmittel zu waschen, nochmals zu spülen und zu trocknen.

35. Verfahren zum Färben nach einem der Ansprüche 31 bis 34, **dadurch gekennzeichnet, dass** die Zusammensetzung A oder A1 und / oder die Zusammensetzung B1 mindestens ein kationisches oder amphoteres Polymer und mindestens einen grenzflächenaktiven Stoff enthält.

36. Vorrichtungen mit zwei Abteilungen oder "Kit" für die Direktfärbung von Keratinfasern und insbesondere menschlichen Keratinfasern wie Haaren, **dadurch gekennzeichnet, dass** eine Abteilung eine Zusammensetzung mit mindestens einem Direktfarbstoff und eine weitere Abteilung eine Zusammensetzung mit mindestens einem Polymer mit Aminoplat-Ether-Gerüst nach einem der Ansprüche 1 bis 12 enthält.

37. Vorrichtung mit zwei Abteilungen oder "Kit" für die aufhellende Direktfärbung von Keratinfasern und insbesondere menschlichen Keratinfasern wie Haaren, **dadurch gekennzeichnet, dass** eine Abteilung eine Zusammensetzung A1, die in einem zum Färben geeigneten Medium mindestens einen Direktfarbstoff enthält, und eine weitere Abteilung eine Zusammensetzung B1 enthält, die ein Oxidationsmittel enthält, wobei mindestens ein Polymer mit Aminoplast-Ether-Grundgerüst in der Zusammensetzung A1 oder in der Zusammensetzung B1 oder in beiden Zusammensetzungen A1 und B1 enthalten ist.

## Claims

1. Composition for the direct dyeing of keratin fibres, in particular human keratin fibres such as the hair, comprising, in a medium that is suitable for dyeing, at least one direct dye, **characterized in that** it also contains at least one thickening polymer with an aminoplast-ether skeleton.

2. Composition according to Claim 1, **characterized in that** the aminoplast-ether polymer(s) is (are) chosen from those containing at least one unit of structure (I) below: 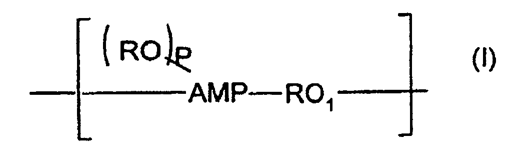 in which:
AMP is an aminoplast residue with alkylene units,
R denotes a hydrogen atom, a C₁-C₄ alkyl radical or a C₁-C₄ acyl radical,
RO₁ is a divalent alkyleneoxy residue,
p denotes a positive integer,
the group(s) OR being linked to the alkylene units of the AMP residue.

3. Composition according to Claim 1 or 2, **characterized in that** the polymer(s) with an aminoplast-ether skeleton is (are) chosen from those containing at least one unit of structure (II) below: 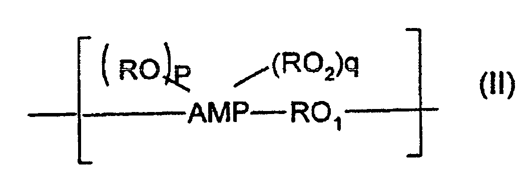 in which:
AMP, R, RO₁ and p have the same meaning as in Claim 2,
RO₂ is hydrophobic group other than RO linked to AMP via a hetero atom and comprising at least two carbon atoms, and
- q is a positive integer.

4. Composition according to Claim 3, **characterized in that** the aminoplast-ether polymer(s) is (are) chosen from the polymers of structure (III) or (IIIa) below: 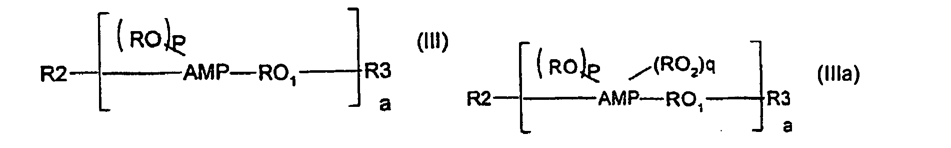 in which:
AMP, R, RO₁, RO₂, p and q have the same meaning as in Claims 2 and 3,
R2 or R3, which may be identical or different, represent an end group that can denote a hydrogen atom,
a group RO₁H, a group RO₂H, a group AMP (OR) p or any monofunctional group such as alkyl, cycloalkyl, aryl, aralkyl, alkylaryl, alkyloxyalkyl, aryloxyalkyl or cycloalkoxyalkyl,
a being a number greater than 1 and preferably greater than 2.

5. Composition according to any one of Claims 2 to 4, **characterized in that** the aminoplast residues bearing the groups OR thereof are chosen from those of structures (IV) to (XV) below: 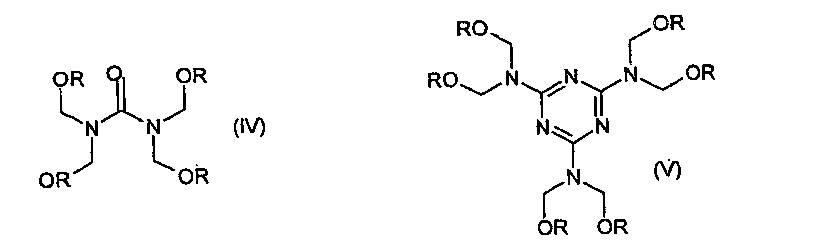 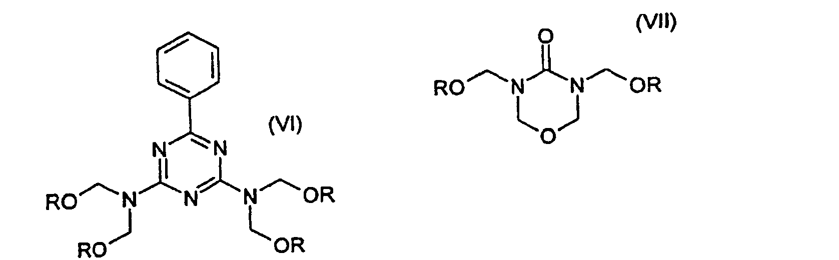 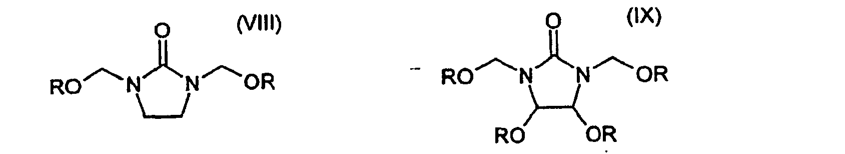 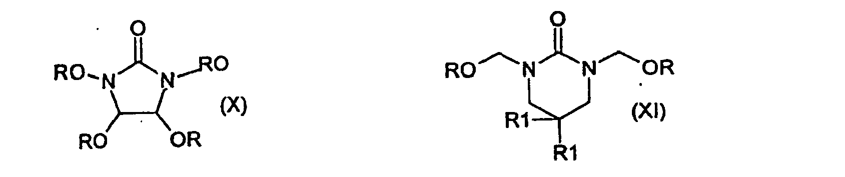 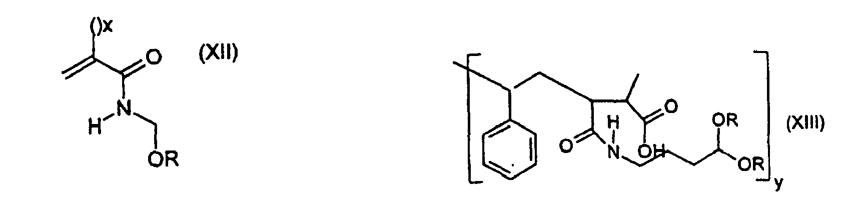 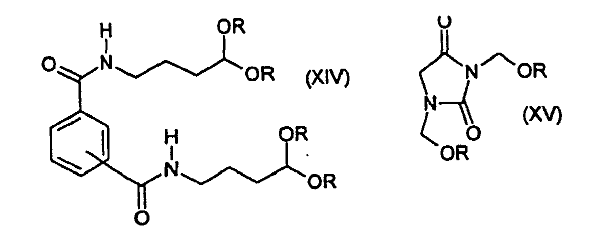
R having the same meaning as in Claim 2,
R1 denotes C₁-C₄ alkyl,
y is a number at least equal to 2,
x denotes 0 or 1.

6. Composition according to any one of Claims 2 to 4, **characterized in that** the aminoplast residues bearing the groups OR thereof are chosen from those of structure (XVI) below: 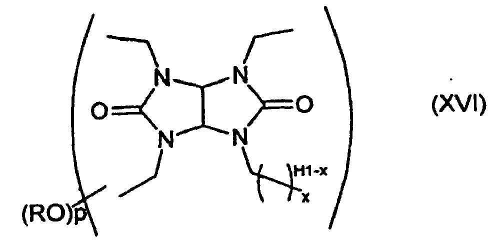 in which R, p and x have the same meanings as in Claims 2 and 5.

7. Composition according to any one of Claims 2 to 6, **characterized in that** the alkyleneoxy residues are those corresponding to the diols of general formula (XVII) below:
HO-(ZO)y-(Z1(Z20)w)t-(Z'O)y'-Z3OH (XVII),
y and y' being numbers ranging from 0 to 1000,
t and w being numbers ranging from 0 to 10,
Z, Z', Z2 and Z3 are C₂-C₄ alkylene radicals and preferably radicals -CH2-CH(Z4)- and -CH2-CH(Z4)-CH2-,
Z1 being a linear or cyclic, branched or unbranched, aromatic or nonaromatic radical optionally comprising one or more hetero atoms containing from 1 to 40 carbon atoms,
Z4 denoting a hydrogen atom or a C₁-C₄ alkyl radical or a C₁-C₃ acyl radical, it being understood that at least one of the radicals Z4 of the radicals Z, Z', Z2 and Z3 is other than acyl.

8. Composition according to Claim 7, **characterized in that** the radical Z4 denotes a hydrogen atom or a methyl radical.

9. Composition according to Claims 7 and 8, **characterized in that** t = 0 and that Z, Z' and Z3 denote -CH2CH2-, at least one from among y and y' being other than 0.

10. Composition according to any one of the preceding claims, **characterized in that** the polymers with an aminoplast-ether skeleton are chosen from the following products:
PEG-180/Octoxynol-40/TMMG Copolymer,
PEG-180/Laureth-50/TMMG Copolymer,
Polyether-1.

11. Composition according to any one of Claims 1 to 10, **characterized in that** the polymers with an aminoplast-ether skeleton are used in an amount that can range from 0.01% to 10% by weight relative to the total weight of the composition.

12. Composition according to Claim 11, **characterized in that** the polymers with an aminoplast-ether skeleton are used in an amount that can range from 0.1% to 5% by weight relative to the total weight of the composition.

13. Composition according to any one of the preceding claims, **characterized in that** the direct dye is chosen from neutral, acidic or cationic nitrobenzene direct dyes, neutral, acidic or cationic azo direct dyes, neutral, acidic or cationic quinone direct dyes and in particular anthraquinone direct dyes, azine direct dyes, triarylmethane direct dyes, indoamine direct dyes and natural direct dyes.

14. Composition according to any one of the preceding claims, **characterized in that** the direct dye(s) is (are) present in concentrations ranging from 0.001% to 20% and preferably from 0.005% to 10% by weight relative to the total weight of the composition.

15. Composition according to any one of the preceding claims, **characterized in that** it also contains at least one cationic or amphoteric polymer.

16. Composition according to Claim 15, **characterized in that** the cationic polymer is a poly(quaternary ammonium) polymer consisting of repeating units corresponding to formula (W) below:

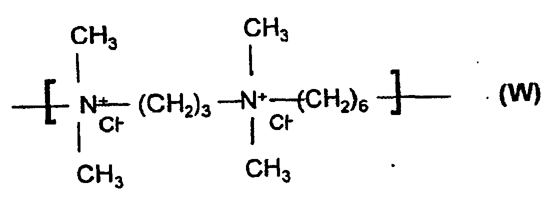

17. Composition according to Claim 15, **characterized in that** the cationic polymer is a poly(quaternary ammonium) polymer consisting of repeating units corresponding to formula (U) below:

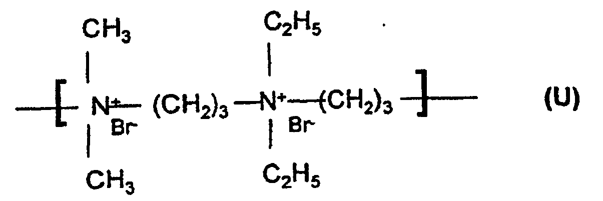

18. Composition according to Claim 15, **characterized in that** the amphoteric polymer is a copolymer comprising at least, as monomers, acrylic acid and a dimethyldiallylammonium salt.

19. Composition according to any one of Claims 15 to 18, **characterized in that** the cationic or amphoteric polymer(s) represent(s) from 0.01% to 10%, preferably from 0.05% to 5% and even more preferably from 0.1% to 3% by weight relative to the total weight of the composition.

20. Composition according to any one of the preceding claims, **characterized in that** it contains at least one surfactant chosen from anionic, cationic, nonionic and amphoteric surfactants.

21. Composition according to Claim 20, **characterized in that** the surfactants represent 0.01% to 40% and preferably 0.1% to 30% by weight relative to the total weight of the composition.

22. Composition according to any one of the preceding claims, **characterized in that** it contains at least one additional thickener.

23. Composition according to Claim 22, **characterized in that** the additional thickener is a cellulose derivative, a guar derivative, a gum of microbial origin or a synthetic thickener.

24. Composition according to Claim 23, **characterized in that** the additional thickener(s) represent(s) 0.01% to 10% by weight relative to the total weight of the ready-to-use composition.

25. Composition according to any one of the preceding claims, **characterized in that** it has a pH ranging from 2 to 12.

26. Ready-to-use composition according to any one of the preceding claims, **characterized in that** it also contains an oxidizing agent.

27. Composition according to Claim 26, **characterized in that** the oxidizing agent is chosen from hydrogen peroxide, urea peroxide, alkali metal bromates or ferricyanides, persalts, and redox enzymes optionally with their respective donor or cofactor.

28. Composition according to Claim 27, **characterized in that** the oxidizing agent is hydrogen peroxide.

29. Composition according to Claim 28, **characterized in that** it is an aqueous hydrogen peroxide solution whose titre ranges from 1 to 40 volumes.

30. Composition according to any one of Claims 26 to 29, **characterized in that** it has a pH of greater than 7 and preferably greater than 8.

31. Process for the direct dyeing of keratin fibres, and in particular of human keratin fibres such as the hair, **characterized in that** it consists in applying to the fibres a dye composition A as defined in any one of Claims 1 to 25.

32. Process for the lightening direct dyeing of keratin fibres, and in particular of human keratin fibres such as the hair, **characterized in that** it consists in applying to the fibres a composition resulting from the mixing, at the time of use, of a composition A1 containing, in a medium that is suitable for dyeing, at least one direct dye, and of an oxidizing composition B1 containing an oxidizing agent, the composition A1 and/or the composition B1 containing at least one polymer with an aminoplast-ether skeleton as defined in any one of Claims 1 to 12.

33. Process according to Claim 31 or 32, **characterized in that** it consists in applying to the wet or dry keratin fibres the ready-to-use composition A or the composition prepared extemporaneously at the time of use from the compositions A1 and B1, leaving it to act for an exposure time ranging from 1 to 60 minutes approximately, and preferably from 10 to 45 minutes, rinsing the fibres and then optionally washing them with shampoo, followed by rinsing them again and drying them.

34. Process for the lightening direct dyeing of keratin fibres, and in particular of human keratin fibres such as the hair, **characterized in that** it consists in applying to the wet or dry keratin fibres the ready-to-use composition prepared extemporaneously at the time of use from a composition A' comprising at least one direct dye, but without polymer with an aminoplast-ether skeleton defined in Claims 1-12, another composition A*''* containing at least one polymer with an aminoplast-ether skeleton defined according to Claims 1 to 12, and an oxidizing composition B, leaving it to act for an exposure time ranging from 1 to 60 minutes approximately and preferably from 10 to 45 minutes, rinsing the fibres and then optionally washing them with shampoo, followed by rinsing them again and drying them.

35. Dyeing process according to any one of Claims 31 to 34, **characterized in that** composition A or A1 and/or composition B1 contain at least one cationic or amphoteric polymer and at least one surfactant.

36. Two-compartment device or kit for the direct dyeing of keratin fibres, and in particular of human keratin fibres such as the hair, **characterized in that** one compartment contains a composition containing at least one direct dye, and another compartment contains a composition comprising at least one polymer with an aminoplast-ether skeleton as defined in any one of Claims 1 to 12.

37. Two-compartment devices or kits for the lightening direct dyeing of keratin fibres, and in particular of human keratin fibres such as the hair, **characterized in that** one compartment contains a composition A1 containing, in a medium that is suitable for dyeing, at least one direct dye, and another compartment contains a composition B1 containing an oxidizing agent, at least one polymer with an aminoplast-ether skeleton being present in composition A1 or in composition B1, or in each of the compositions A1 and B1.
